Europäisches Patentamt

European Patent Office

Office européen des brevets

(19)

(11) Veröffentlichungsnummer : **0 290 736 B1**

# (12) EUROPÄISCHE PATENTSCHRIFT

(45) Veröffentlichungstag der Patentschrift :
05.06.91 Patentblatt 91/23

(51) Int. Cl.$^5$ : **A61F 2/38**

(21) Anmeldenummer : **88103551.3**

(22) Anmeldetag : **08.03.88**

(54) In der Tibia verankerbarer metallener Verankerungsteil für eine Kniegelenk-Endoprothese.

(30) Priorität : **15.05.87 CH 1888/87**

(43) Veröffentlichungstag der Anmeldung :
**17.11.88 Patentblatt 88/46**

(45) Bekanntmachung des Hinweises auf die
Patenterteilung :
**05.06.91 Patentblatt 91/23**

(84) Benannte Vertragsstaaten :
**AT DE FR GB IT**

(56) Entgegenhaltungen :
**EP-A- 0 135 319**
**DE-A- 3 429 157**
**US-A- 3 774 244**
**US-A- 4 158 684**

(73) Patentinhaber : **GEBRÜDER SULZER**
**AKTIENGESELLSCHAFT**
**Zürcherstrasse 9**
**CH-8401 Winterthur (CH)**

(72) Erfinder : **Karpf, Kurt**
**Alte Strasse 175**
**CH-4718 Holderbank (CH)**
Erfinder : **Böhler, N., Prof. Dr. med.**
**Promenade 6**
**A-4020 Linz (AT)**

**Beschreibung**

Die Erfindung betrifft einen in der Tibia verankerbaren metallenen Verankerungsteil für eine Kniegelenk-Endoprothese, der als Träger für eine Lager- und Gleitfläche aus Kunststoff dient.

Tibiateile von Kniegelenk-Prothesen werden bisher zementfrei mit Hilfe von Zapfen (EP-A-151 724) oder Dübeln (EP-A-170 779) in der Tibia verankert, wobei die Zapfen oder Dübel in Bohrungen eingesteckt werden, die sich in Richtung der Längsachse von der Tibia-Kondylenfläche in den Knochen hinein erstrecken.

Bei nur geringfügig zerstörten Kniegelenken, bei denen unter Umständen nur ein Teilersatz vorgenommen wird, ergeben sich mit den bekannten Verankerungssystemen Schwierigkeiten ; denn in diesem Fall soll nämlich der gesamte Bandapparat mit den beiden Seiten- und den beiden Kreuzbändern erhalten bleiben, wobei angestrebt wird, dass auch intraoperativ keine Bandablösungen vorgenommen werden müssen.

. Bei intaktem Bandapparat können jedoch Femur und Tibia nur beschränkt gegeneinander verschoben und in Richtung der Längsachse distrahiert werden. Besonders tibiaseitig ist es daher kaum möglich, die für die geschilderten zementlosen Verankerungen notwendigen Bohrungen in die Tibia einzubringen.

Aufgabe der Erfindung ist es daher, eine Tibia-Prothese zu schaffen, die im Knochen verankert werden kann, ohne dass der Bandapparat angetastet werden muss.

Diese Aufgabe wird mit der Erfindung dadurch gelöst, dass das dem Knochen zugewandte, in Richtung ventral/dorsal im wesentlichen ebene Tibia-Plateau von lateral bzw. medial zur Mitte des Tibia-Knochens hin geneigt ist und sich in Richtung ventral/dorsal erstrekkende, hohle Verankerungsschienen aufweist, in die Fixierungselemente einschiebbar sind.

Die neue Prothese ist von ventral nach dorsal wie in eine Schublade in den Unterschenkel einschiebbar. Ihre Fixierung auf der Tibia erfolgt mit Hilfe der beiden Verankerungsschienen. Da diese von ventral nach dorsal verlaufen, werden alle Lehren und Instrumente ebenfalls von ventral nach dorsal in den Tibiaknochen eingebracht, ohne dass der Bandapparat angetastet werden muss. Die in den Knochen eingeschobene Prothese wird zusätzlich gegen Verschiebungen durch die Fixierungselemente gesichert.

Eine konstruktiv vorteilhafte Ausführungsform für die Verankerungsschienen ergibt, wenn die Veranke-rungsschienen als kreiszylindrische Rohre ausgebil-det sind, an deren Kreisquerschnitt ein Abschnitt durch das Tibia-Plateau angeschnitten ist ; denn die über dem Aequator der Kreisquerschnitte gelegenen Bereich des Umfanges wirken als Hinterschneidungen, die eine Bewegung der fixierten Prothese in Richtung der Längsachse verhindern.

Als Fixierungen gegenüber Verschiebungen in Richtung ventral/dorsal haben sich Fixierungselemente bewährt, die als Nocken einer im Verankerungsrohr exzentrisch und drehbar gelagerten Welle ausgebildet sind und durch Schlitze im Mantel des Verankerungsrohres in die Spongiosa eindrehbar sind. Dabei kann die Drehung der Welle durch einen Anschlag begrenzt sein. Eine andere konstruktiv vorteilhafte Art der Fixierungselemente sind mit Innengewinde versehene Sprengringe und ein mindestens teilweise konischer Schraubenbolzen.

Bevorzugt kann der neue Verankerungsteil als halbseitige Teilprothese ausgebildet sein. Ist in in diesem Fall bezüglich einer senkrecht zur ventral/dorsal-Richtung stehenden Mittelebene spiegelsymmetrisch, so lässt es sich wahlweise als mediale und als laterale Teilprothese verwenden.

Bei der Implantation empfiehlt sich beispielsweise folgendes Vorgehen :

Mit entsprechenden Instrumenten werden – in vorbestimmter Höhe und im richtigen Winkel – zunächst Verankerungsbohrungen für die Aufnahme der Verankerungsschienen in den Tibiakopf gebohrt.

Mit einem Meissel, welcher in den Verankerungsbohrungen geführt ist, wird dann überschüssiges Knochenmaterial entfernt. Die Verankerungsbohrungen sind dabei über dem Aequator so etwas angeschnitten, dass ein in die Verankerungsbohrung eingebrachtes Fixierungselement noch allseitig ausreichend geführt ist.

Die Schnittfläche am Knochen ist identisch mit der Auflagefläche auf dem Tibiakopf. Bei einer halbseitigen Teilprothese sind dabei zwei tragende Abstützfläche für das Implantat vorhanden : Einmal die grosse, von lateral nach medial oder umgekehrt schräge Abstützfläche und zum zweiten eine kleinere Stützfläche, die sich an der Eminentia abstützt. Die beiden Abstützflächen stehen dabei vornehmlich in einem Winkel von 75° bis 110° zueinander. Der Uebergang von der grossen Abstützfläche zu der fast senkrechten Fläche kann dabei sowohl als Kante ausgebildet sein, als auch durch einen Radius erfolgen.

Der neue Verankerungsteil besteht aus einem der bekannten metallischen Prothesenwerkstoffe. Er kann gegossen oder geschmiedet sein. Darüberhinaus kann seine Kontaktfläche zum Knochen ebenfalls in bekannter Weise mit einer gewebefreundlichen Beschichtung oder Struktur versehen sein. Die Lager- und Gleitfläche gegenüber einer vorzugsweise ebenfalls metallischen Femurprothese ist aus Polyäthylen der für Implantate üblichen Spezifikationen gefertigt. Dabei kann der Kunststoffteil bereits während der Fabrikation mit dem metallenen Verankerungsteil zu einer Einheit verbunden sein und als steriles Implantat geliefert werden. Es ist aber auch möglich, Polyäthylenteil und Verankerungsteil mit Schnappverschlüssen zu versehen, so dass die endgültige Verbindung erst intraoperativ vorgenommen wird.

Im folgenden wird die Erfindung anhand von Ausführungsbeispielen im Zusammenhang mit der Zeichnung näher erläutert.

Fig. 1 ist in Richtung ventral/dorsal eine Ansicht einer ersten Ausführungsform der Verankerungsteils für eine Kniegelenkprothese, die beide, d.h. die mediale und die laterale, Tibia-Kondyle ersetzt ;

Fig. 2 gibt in gleicher Darstellung eine zweite Ausführungsform wieder, die Verankerungsteil für eine Teilprothese ist ;

Fig. 3 zeigt den Verankerungsteil nach Fig. 2 – wiederum in einer ventral/dorsal-Ansicht – in einem Tibia-Knochen implantiert ;

Fig. 4 ist eine Aufsicht auf Fig. 3 ;

Fig. 5 stellt als Detail aus Fig. 3 ein Fixierungselement dar ;

Fig. 6 ist eine Ansicht von Fig. 5 von unten ;

Fig. 7 gibt als Detail von Fig. 1 ein zweites Ausführungsbeispiel für ein Fixierungselement in einer Ansicht von distal auf das Tibia-Plateau teilweise im Schnitt wieder.

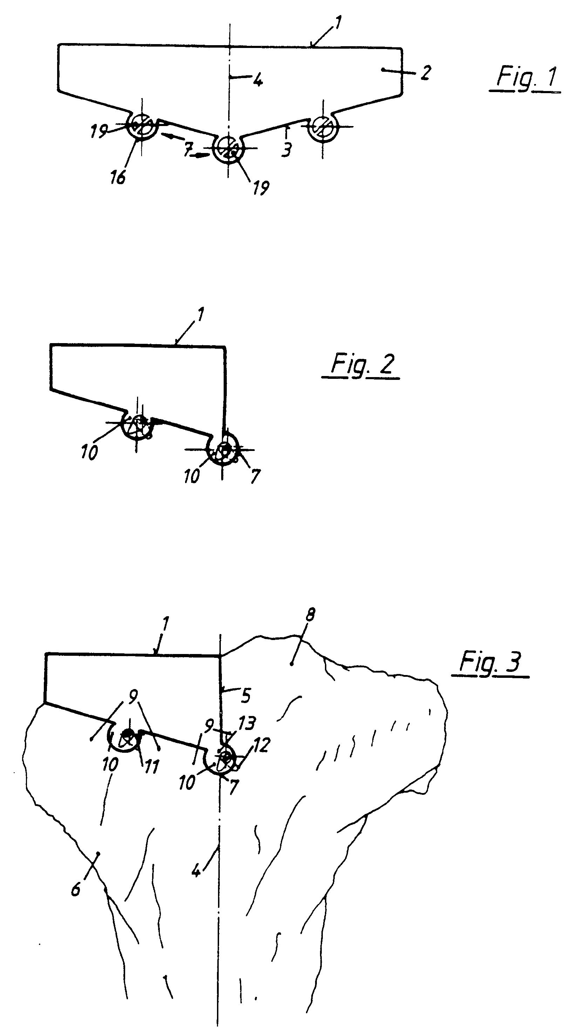

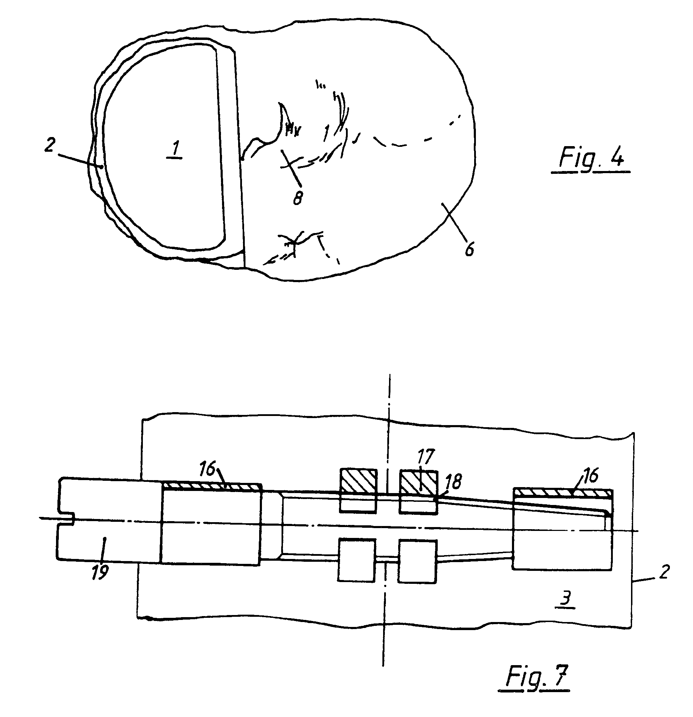

Die tibiale Lagerfläche 1 (Fig. 1) – eine Aufsicht auf sie ist in Fig. 4 dargestellt – des Verankerungsteils 2 dient als "Fundament" für einen nicht gezeigten Kunststoffkörper, der die eigentliche Lager- und Gleitfläche für eine ebenfalls nicht gezeigte Prothese einer oder beider Femurkondylen bildet. Während die tibiale Lagerfläche 1 des Verankerungsteils 2, der als Teilprothese für einen Ersatz der lateralen oder der medialen Tibia-Gleitfläche dienen oder den ganzen Tibiakopf ersetzen kann, im wesentlichen horizontal verläuft, ist sein Tibia-Plateau 3 als zur Lagerfläche schräge Fläche ausgebildet, die jeweils vom lateralen der medialen Rand zur Mittelachse 4 der Tibia 6 (Fig. 3) hin nach distal abfällt.

Bei als Teilprophesen dienenden Ausführungsformen (Fig. 2) bildet eine in der Achse 4 verlaufende Begrenzung 5 des Verankerungsteils 2 eine zusätzliche Stützfläche, die sich im operativ entsprechend vorbereiteten Tibiaknochen an der Eminentia 8 abstützt und als zur Hauptabstützfläche des Tibia-Plateaus 3 zusätzliche Verankerungsfläche dient, an die Knochengewebe anwachsen kann.

Als eigentliche Verankerungselemente, die ein Aufschieben des Verankerungsteils 2 auf den Tibiakop von ventral nach dorsal ermöglichen, sind an das Tibia-Plateau 3 Verankerungsschienen 7 angesetzt, die schubladenartig in entsprechend operativ geschaffene "Gleitbahnen" in der Tibia 6 eingeschoben werden.

Die Verankerungsschienen 7 sind rohrförmig, d.h. sie haben auf ihrer ganzen Länge von ventral nach dorsal einen konstanten Aussendurchmesser. In den gezeigten Ausführungsformen ist der Querschnitt kreisförmig, wobei ein Abschnitt des Kreises von der Fläche des Tibia-Plateaus 3 angeschnitten ist. Dadurch entstehen Hinterschneidungen 9, die ein "Abheben" des Verankerungsteils 2 von der Knochenfläche nach proximal in Richtung der Tibia-Achse verhindern.

Für die Fixierung der "Schublade" gegen ein Oeffnen sind in Verbindung mit den Verankerungsschienen 7 Fixierungselemente vorgesehen. Dafür seien im folgenden zwei Beispiele beschrieben.

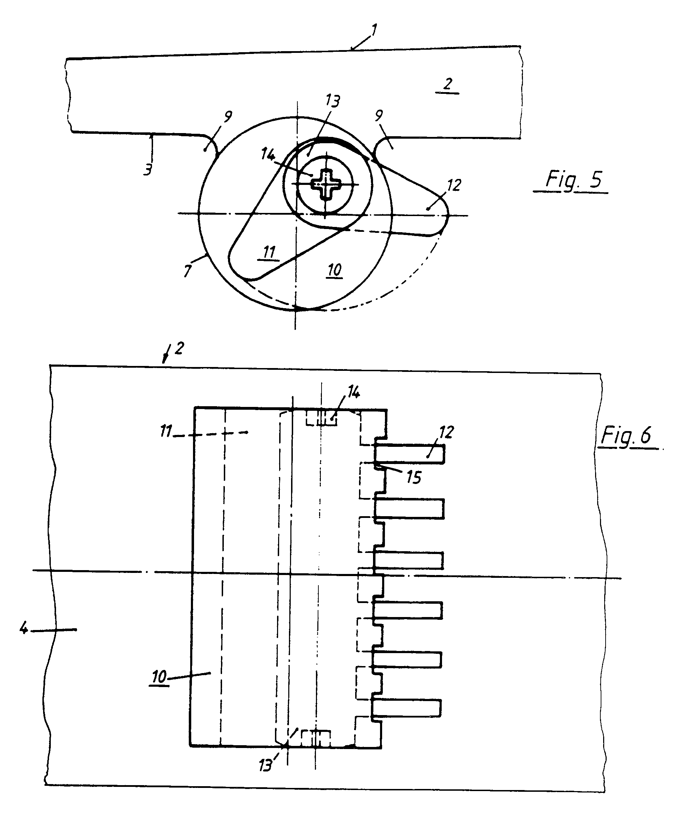

Bei der einen, in Fig. 2, 3, 5 und 6 gezeigten Konstruktion erfolgt die Fixierung mit Hilfe einer sicherheitsschlossartigen Verriegelung. Die Verankerungsschiene 7 enthält daher einen Schlosszylinder 10, in dem exzentrisch eine im Querschnitt nockenwellenförmige Bohrung 11 vorhanden ist. In dieser Bohrung 11 sitzt eine mit Nocken 12 versehene Welle 13, die an ihren Stirnseiten kreuzkopf- schraubenartige Vertiefungen 14 für das Einsetzen eines Schlüssels hat.

Entsprechend der Anzahl der Nocken 12 weisen der Zylinder 11 und das Verankerungsrohr 7 Schlitze auf, in denen die Nocken 12 aus dem Zylinder 11 und dem Rohr 7 herausgeschwenkt werden können. Die Nocken 12 dringen beim Herausschwenken in weiches spongioses Gewebe ein und schaffen so eine Verriegelung des Verankerungsteils 2 gegen Verschiebungen ventral/dorsal. Durch Anschlagen der Nocken 12 am "Ende" der Schlitze 15 wird die Drehbewegung dabei begrenzt, so dass ein "Ueberdrehen" vermieden wird.

In Fig. 1 und 7 ist eine etwas einfachere Konstruktion von Fixierungselementen gezeigt. Hier besteht die Verankerungsschiene aus zwei rohrförmigen Hülsen 16 ; für die Fixierung des Verankerungsteils 2 sind zwei infolge eines nicht dargestellten Schlitzes aufweitbare Sprengringe 17 vorhanden, die mit einem Innengewinde 18 versehen sind. In das Gewinde 18 ist ein an seinem Ende konischer Schraubenbolzen 19 einschraubbar, durch dessen Einschrauben die Sprengringe 17 aufgeweitet und in das spongiose Gewebe eingepresst werden.

Hülsen 16 und Sprengringe 17 sind beispielsweise mit dem Verankerungsteil 2 aus einem Stück gefertigt, was den Vorteil hat, dass die Schwierigkeiten ihrer genauen Ausrichtung, die bei einer nachträglichen Befestigung, beispielsweise durch Schweissen, auftreten können, umgangen werden. Selbstverständlich ist jedoch ein Anschweissen oder Anlöten der Hülse 16 und der Sprengringe 17 an den Verankerungsteil 12 grundsätzlich ebenfalls möglich.

Um Teilprothesen sowohl lateral als auch medial verwenden zu können, sind sowohl der Verankerungsteil 2 als auch die Verankerungsschienen 7 bzw. 16 und die Fixierungselemente 10 bis 14 bzw. 17 spiegelsymmetrisch zu einer senkrecht zur ventral/dorsal-Richtung verlaufenden Mittelebene 20 ausgebildet.

## Ansprüche

1. In der Tibia verankerbarer metallener Verankerungsteil für eine Kniegelenk-Endoprothese, der als Träger für eine Lager- und Gleitfläche aus Kunststoff dient, dadurch gekennzeichnet, dass das dem Knochen (6) zugewandte, in Richtung ventral/dorsal im wesentlichen ebene Tibia-Plateau (3) von lateral bzw. medial zur Mitte des Tibia-Knochens (6) hin geneigt ist und sich in Richtung ventral/dorsal erstreckende hohle Verankerungsschienen (7, 16) aufweist, in die Fixierungselemente (10 bis 14, 17, 19) einschiebbar sind.

2. Verankerungsteil nach Anspruch 1, dadurch gekennzeichnet, dass die Verankerungsschienen (7, 16) als kreiszylindrische Rohre ausgebildet sind, von deren Kreisquerschnitt ein Abschnitt durch das Tibia-Plateau (3) angeschnitten ist.

3. Verankerungsteil nach Anspruch 2, dadurch gekennzeichnet, dass die Fixierungselemente die Nocken (12) einer im Rohr (7) exzentrisch und drehbar gelagerten Welle (13) sind, die durch Schlitze (15) im Mantel des Verankerungsrohres (7) in die Spongiosa eindrehbar sind.

4. Verankerungsteil nach Anspruch 3, dadurch gekennzeichnet, dass die Drehung der Welle (13) durch einen Anschlag begrenzt ist.

5. Verankerungsteil nach Anspruch 2, dadurch gekennzeichnet, dass die Fixierungselemente aus mit Innengewinden (18) versehenen Sprengringen (17) und einem mindestens teilweise konischen Schraubenbolzen (19) bestehen.

6. Verankerungsteil nach Anspruch 1, dadurch gekennzeichnet, dass er als halbseitige Teilprothese ausgebildet ist.

7. Verankerungsteil nach Anspruch 6, dadurch gekennzeichnet, dass er bezüglich einer senkrecht zur ventral/dorsal-Richtung stehenden Mittelebene (20) spiegelsymmetrisch ist.

## Claims

1. A metal fixing member for a knee joint endoprosthesis, such member being fixable in the tibia and serving as carrier or support for a plastics bearing and rubbing surface, characterised in that the tibia plateau (3) which is near the bone (6) and which is substantially plane ventrally to dorsally is inclined from the lateral or medial direction towards the centre of the tibia bone (6) and has hollow fixing rails (7, 16) which extend ventrally to dorsally and into which the fixing elements (10-14, 17, 19) can be introduced.

2. A fixing member according to claim 1, characterised in that the fixing rails (7, 16) are in the form of cylindrical tubes and the tibia plateau (3) cuts off a part of their circular cross-section.

3. A fixing member according to claim 2, characterised in that the fixing elements are the protuberances (12) of a shaft (13) which is mounted eccentrically and rotatably in the tube (7), the protuberances (12) being adapted to be turned into the spongey bone tissue through slots (15) in the generated surface of the fixing tube (7).

4. A fixing member according to claim 3, characterised in that rotation of the shaft (13) is limited by a stop.

5. A fixing member according to claim 2, characterised in that the fixing elements are in the form of circlips (17) having internal screwthreads (18) and of a screwthreaded fastener (19) which is at least partly conical.

6. A fixing member according to claim 1, characterised in that it is in the form of a half-side partial prosthesis.

7. A fixing member according to claim 6, characterised in that it is in symmetrical laterally inverted relationship in respect of a centre-plane (20) extending perpendicularly to the ventral-to-dorsal direction.

## Revendications

1. Pièce d'ancrage métallique à ancrer dans le tibia pour une endoprothèse de l'articulation du genou, qui sert de support à une surface d'appui et de glissement en matière plastique, caractérisée en ce que le plateau de tibia (3), à peu près plan en direction ventrale/dorsale, tourné vers l'os (6), a une inclinaison latérale ou médiale en direction du milieu du tibia (6) et présente des glissières d'ancrage (7, 16) creuses s'étendant en direction ventrale/dorsale, dans lesquelles peuvent être enfilés des éléments de fixation (10 à 14, 17, 19).

2. Pièce d'ancrage selon la revendication 1, caractérisée en ce que les glissières d'ancrage (7, 16) sont des tubes cylindriques circulaires dont une partie de la section transversale circulaire est coupée par le plateau de tibia (3).

3. Pièce d'ancrage selon la revendication 2, caractérisée en ce que les éléments de fixation sont les cames (12) d'un arbre (13) monté excentré et tournant dans le tube (7) et qui peuvent pénétrer en tournant dans le tissu spongieux, par des fentes (15) pratiquées dans l'enveloppe du tube d'ancrage (7).

4. Pièce d'ancrage selon la revendication 3, caractérisée en ce que la rotation de l'arbre (13) est limitée par une butée.

5. Pièce d'ancrage selon la revendication 2, caractérisée en ce que les éléments de fixation sont constitués par des bagues élastiques (17) pourvues de taraudages (18) et par un boulon (19) au moins en partie conique.

6. Pièce d'ancrage selon la revendication 1, caractérisée en ce qu'elle se présente sous la forme d'une prothèse partielle semi-latérale.

7. Pièce d'ancrage selon la revendication 6, caractérisée en ce qu'elle présente une symétrie spéculaire par rapport à un plan médian (20), perpendiculaire à la direction ventrale/dorsale.

Fig. 1

Fig. 2

Fig. 3

*Fig. 4*

*Fig. 7*

Fig. 5

Fig. 6